(19)

Europäisches Patentamt

European Patent Office

Office européen des brevets

(11) **EP 4 708 306 A2**

(12) **EUROPEAN PATENT APPLICATION**

(43) Date of publication:
**11.03.2026 Bulletin 2026/11**

(21) Application number: **26151818.7**

(22) Date of filing: **22.10.2024**

(51) International Patent Classification (IPC):
**G16C 20/70** (2019.01)

(52) Cooperative Patent Classification (CPC):
**G16C 20/70; G16C 60/00;** G16C 20/30; Y02E 60/10

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **25.10.2023 KR 20230143658**

(62) Document number(s) of the earlier application(s) in accordance with Art. 76 EPC:
**24207978.8 / 4 546 354**

(71) Applicant: **LG Management Development Institute**
**Seoul 07336 (KR)**

(72) Inventors:
• **Park, Changyoung**
**07982 Seoul (KR)**

• **Lee, Jaewan**
**07792 Seoul (KR)**
• **Yang, Hongjun**
**07788 Seoul (KR)**
• **Han, Sehui**
**07665 Seoul (KR)**

(74) Representative: **BCKIP Part mbB**
**MK1**
**Landsbergerstraße 98, 3.Stock**
**80339 München (DE)**

Remarks:
This application was filed on 14.01.2026 as a divisional application to the application mentioned under INID code 62.

(54) **DEVICE, METHOD AND PROGRAM FOR ACQUIRING FEATURE DATA FOR MATERIAL COMPOSITION INFORMATION BASED ON ARTIFICIAL INTELLIGENCE**

(57)    A system, device, method, and program for acquiring feature data for material composition information based on artificial intelligence are disclosed. The system may include a memory configured to store a first artificial intelligence (AI) model configured to output first feature data for composition information of a material and a second AI model configured to output second feature data for structure information of the material; and a processor configured to learn the first AI model and the second AI model. The processor may be configured to learn the first AI model based on the second feature data for the structure information of the material output by the second AI model, and/or to learn the second AI model based on the first feature data for the composition information of the material output by the first AI model.

FIG. 3

EP 4 708 306 A2

## Description

## BACKGROUND

The present disclosure generally relates to a device, method, and program for acquiring feature data for material composition information. More specifically, some exemplary embodiments of the present disclosure relate to a device, method, and program for acquiring feature data for material composition information based on artificial intelligence.

As lithium-ion battery technology develops, lithium-ion batteries are used in various fields such as electric vehicles and energy storage systems. As a result, a positive electrode material capable of increasing the energy storage capacity and the energy density of a lithium-ion battery has been actively developed, and studied.

Experiments to analyze various types of materials for the development of such a positive electrode material requires a considerable amount of time and cost. However, as the processing ability of computers is improved and databases for material research such as the Open Quantum Materials Database (OQMD) are developed, it becomes possible to quickly conduct research on the molecular structure, crystal structure, and the like of the material by utilizing artificial intelligence.

However, in the field of batteries, research on a positive electrode material using such artificial intelligence (particularly, deep learning artificial intelligence) was not actively progressed because data related to the material of the battery was not sufficient, and it was difficult to acquire related data for structure information among material information of the battery. This has increased the need for methods that can analyze battery materials based on insufficient data.

## SUMMARY

Some embodiments of the present disclosure may provide a device, method, and program for acquiring feature data for material composition information based on artificial intelligence.

The problems to be solved by the present disclosure are not limited to the above-mentioned descriptions, and other problems not mentioned may be clearly understood by those skilled in the art from the following descriptions.

An device for acquiring feature data for composition information of a material according to an aspect of the present disclosure for achieving the above-described technical problem includes: a memory storing a first artificial intelligence (AI) model that outputs first feature data for the composition information of the material and a second AI model that outputs second feature data for the structure information of the material; and a processor configured to execute or learn the first AI model and the second AI model; wherein the processor may be configured to learn the first AI model based on the second feature data for the structure information of the material output by the second AI model, or to learn the second AI model based on the first feature data for the composition information of the material output by the first AI model.

And, the processor learning the first AI model based on the second feature data related to the structural information of the material outputted by the second AI model may involve learning the first AI model to output feature data that has an association with the second feature data.

And, the first feature data and the second feature data have multiple dimensions, and learning the first AI model to output feature data that has an association with the second feature data by the processor may involve adjusting the first feature data to a third feature data in such a way that they have a higher similarity when the first feature data and the second feature data are of the same dimension, thereby enabling the first AI model to be learned to output the adjusted third feature data.

And, the third feature data and fourth feature data have multiple dimensions, and the processor may be configured to: adjust the second feature data to the fourth feature data such that, if the second feature data and the third feature data have the same dimension, they have a higher similarity, and to learn the second AI model to output the adjusted fourth feature data, and adjust the third feature data to fifth feature data such that, if the third feature data and the fourth feature data have the same dimension, they have a higher similarity, and to re-learn the first AI model to output the adjusted fifth feature data.

And, the first AI model is any one of Multi-Layer Perceptron (MLP), Graph Neural Network (GNN), or Transformer Encoder, and the second AI model may be a GNN.

And, the material is one of multiple materials comprising lithium oxide, and the processor may be configured to learn the first AI model and the second AI model based on a specific material, and then re-learn the first AI model and the second AI model based on a material different from the specific material.

And, the first AI model, learned based on the second feature data for the structure information of the material output by the second AI model, may receive as input the composition information of the cathode material of a battery in a charged state and/or the composition information of the cathode material of the battery in a discharged state to acquire feature data used for predicting the average voltage of the battery.

And, the feature data may be a feature vector.

And, according to another embodiment of the present disclosure, a method performed by a device for acquiring feature data for material composition information may include outputting the first feature data by inputting the composition information of the material into the first AI model stored in memory by the processor comprised in the device; outputting the second feature data by inputting the structural information of the material into the second AI model stored in the memory by the processor; and learning the first AI model based on the second feature data related to the structural information of the material outputted by the second AI model, or learning the second AI model based on the first feature data related to the composition information of the material outputted by the first AI model, by the processor.

And, the learning of the first AI model by the processor based on the second feature data related to the structural information of the material outputted by the second AI model may involve learning the first AI model to output feature data that has an association with the second feature data.

And, the first feature data and the second feature data have multiple dimensions, and learning the first AI model to output feature data that has an association with the second feature data by the processor may involve adjusting the first feature data to a third feature data in such a way that they have a higher similarity when the first feature data and the second feature data are of the same dimension, thereby enabling the first AI model to be learned to output the adjusted third feature data.

And, the third feature data and fourth feature data have multiple dimensions, and the present disclosure may further comprise: adjusting the second feature data to the fourth feature data by the processor such that, if the second feature data and the third feature data have the same dimension, they have a higher similarity, and learning the second AI model to output the adjusted fourth feature data, and adjusting the third feature data to fifth feature data by the processor such that, if the third feature data and the fourth feature data have the same dimension, they have a higher similarity, and re-learning the first AI model to output the adjusted fifth feature data.

And, the present disclosure may further comprise: inputting the composition information of the cathode material of a battery in a charged state and/or the composition information of the cathode material of the battery in a discharged state, to the first AI model, learned based on the second feature data for the structure information of the material output by the second AI model; and acquiring feature data used for predicting the average voltage of the battery by the processor.

Furthermore, a computer-readable recording medium storing a computer program for executing the method for implementing the present disclosure may further be provided.

Therefore, according to certain embodiments of the present disclosure, a device, method, and program for acquiring feature data for material composition information based on artificial intelligence may be provided.

In addition, according to some embodiments of the present disclosure, the feature data may be obtained only by the composition information of the material, and the performance of the device, the method, and the program for acquiring the feature data by using the structure information of the material is not significantly different. That is, since various embodiments of the present disclosure are capable of acquiring, extracting, and outputting feature data of a material with similar performance even by using compositional information that is easy to acquire relative data rather than structural information that is difficult to acquire research and testing data on the material may be further facilitated. In particular, some embodiments of the present disclosure may acquire feature data of a positive electrode material of a battery in which related data is insufficient.

The effects of the present disclosure are not limited to the effects mentioned above, and other effects not mentioned may be clearly understood by those skilled in the art from the following description.

**BRIEF DESCRIPTION OF DRAWINGS**

FIG. 1 is a schematic diagram of a system for acquiring feature data for material composition information based on artificial intelligence according to an embodiment of the present disclosure.

FIG. 2 is a block diagram illustrating a device for performing a method for acquiring feature data for artificial intelligence-based material composition information according to an embodiment of the present disclosure.

FIG. 3 is a schematic block diagram illustrating a method of learning an AI model according to an embodiment of the present disclosure.

FIG. 4 is a table for explaining the performance of an AI model according to an embodiment of the present disclosure.

FIG. 5 is a flowchart illustrating a method of learning an AI model according to an embodiment of the present disclosure.

FIG. 6 is a flowchart illustrating a method of learning an AI model according to an embodiment of the present disclosure.

FIG. 7 is a block diagram illustrating a method of predicting or calculating an average voltage of a battery using an AI model, according to an embodiment of the present disclosure.

FIG. 8 is a graph for illustrating performance of a method for extracting feature data of a battery positive electrode material using an AI model according to an embodiment of the present disclosure.

**DETAILED DESCIPTION**

Throughout the present disclosure, the same reference numerals designate the same components. The present disclosure does not describe all elements of the embodiments, and general contents in the technical field of the present disclosure or duplicated content among the embodiments are omitted. The terms "unit, module, member, block" used in the specification may be implemented in software or hardware, and depending on the embodiments, multiple "units, modules, members, blocks" may be implemented as a single component, or a single "unit, module, member, block" may also include multiple components.

Throughout the specification, when a part is described as being "connected" to another part, it includes not only cases where they are directly connected but also cases where they are indirectly connected, which may be connected through a wireless communication network.

Furthermore, when a part is described as "comprising" a certain component, it means that it may further include other components, not excluding other components unless explicitly stated otherwise.

Throughout the specification, when one member is described as being "on" other member, it includes not only cases where the members are in contact but also cases where another member exists between them.

Terms such as "first" and "second" are used to distinguish one component from another, and are not intended to limit the components to those aforementioned by the terms.

Singular expressions include plural expressions unless the context clearly indicates otherwise.

Identification codes used for each step are provided for convenience in description and do not specify the order of the steps, and each step may be carried out in a different order unless a specific order is explicitly described.

The operating principles and embodiments of the present disclosure will be described below with reference to the accompanying drawings.

The term a device for acquiring feature data for material composition information in this specification encompasses various devices capable of performing operations and providing results to users. For example, the device for acquiring feature data for material composition information, according to the present disclosure may include a computer, server device, and portable terminal, or it may take any one of these forms.

Here, the computer may include, for example, a notebook, desktop, laptop, tablet personal computer (PC), or slate PC, equipped with a web browser.

The server device is a server that processes information by communicating with an external device, and may include an application server, a computing server, a database server, a file server, a game server, a mail server, a proxy server, and a web server.

The portable terminal is for example, a wireless communication device ensuring portability and mobility, and may include all kinds of handheld-based wireless communication devices such as Personal Communication System (PCS), Global System for Mobile communications (GSM), Personal Digital Cellular (PDC), Personal Handyphone System (PHS), Personal Digital Assistant (PDA), International Mobile Telecommunication (IMT)-2000, Code Division Multiple Access (CDMA)-2000, W-Code Division Multiple Access (W-CDMA), Wireless Broadband Internet (WiBro) terminal, smart phone, and wearable devices such as watches, rings, bracelets, anklets, necklaces, glasses, contact lenses, or head-mounted devices (HMD).

Hereinafter, embodiments of the present invention will be described in detail with reference to the accompanying figures.

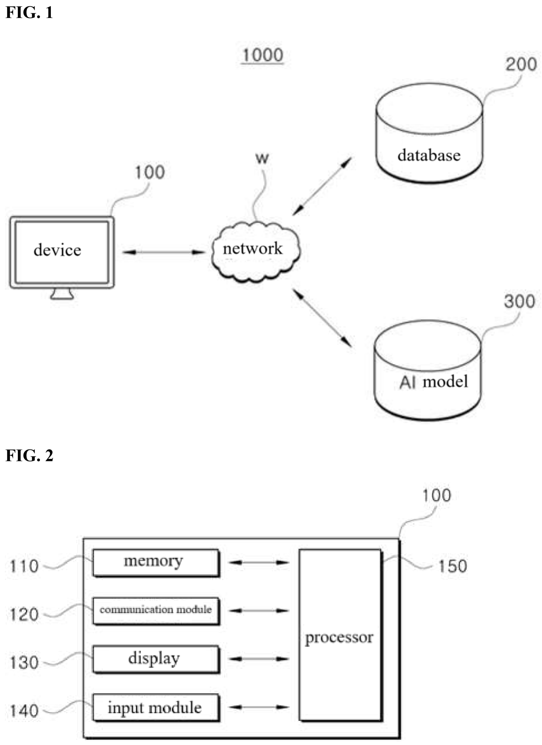

Fig. 1 is a schematic diagram of a system for acquiring feature data for composition information of a material according to an embodiment of the present disclosure.

As shown in FIG. 1, a system 1000 for acquiring feature data for composition information of a material may include a device or terminal 100, a database 200, and an artificial intelligence (AI) model 300.

The device 100, the database 200, and the AI model 300 included in the system 1000 may perform communication via a network W. Here, the network W may include a wired network and a wireless network. For example, the network may include various types of networks such as a local area network (LAN), a metropolitan area network (MAN), and a wide area network (WAN).

The network W may also include a World Wide Web (WWW). However, the network W according to embodiments of the present disclosure is not limited to the above-listed networks, and may include, at least in part, any kinds of networks such as a known wireless data network, a known telephone network, or a known wired or wireless television network.

The device 100 may obtain feature data about composition information of a material based on or using the AI model 300. Here, the AI model 300 may output feature data for composition information of a material, the feature data may include a feature vector as data indicating each feature of the material, and a feature value may be a negative value or a positive value, and may include a value equal to or less than a decimal point in the composition information of the material.

For example, the composition information of a material includes a chemical formula, a composition ratio of an element or an atom, chemical information, and the like, and the chemical information may include various types of molecular information (e.g., a simplified molecular-input line-entry system (SMILES), an international chemical identifier (INCHI), or a self-referencing embedded string (SELFIES)). As an example, the material includes a cathode material of a battery.

The device 100 may calculate or predict various characteristics and features of a material based on the acquired feature data, which may be used to calculate or predict the average voltage of the battery positive electrode material in one embodiment.

The database 200 may store various learning data for learning the AI model 300. In addition, the database 200 may store chemical information, such as the structure or composition of one or more materials, as well as output data produced by the AI model 300. If the learning of the AI model 300 is complete, the database 200 may not be needed in the system 1000.

Fig. 1 illustrates an exemplary embodiment where the database 200 is implemented as a separate device from the device 100. In this case, the database 200 may be connected to the device 100 in a wired and/or wireless manner. However, this is merely one exemplary embodiment, and the database 200 may be implemented as a component of the device 100 or be included in the device 100.

FIG. 1 illustrates an exemplary embodiment where the AI model 300 is connected to the device 100 through the network W (e.g., implemented in a cloud-based manner), but the present disclosure is not limited thereto, and may be implemented as a component in or included in the device 100.

FIG. 2 is a block diagram illustrating a device configured to perform a method of acquiring feature data for composition information of an artificial intelligence-based material according to an embodiment of the present disclosure.

As shown in FIG. 2, the device 100 may include a memory 110, a communication module or communicator 120, a display 130, an input module or input interface 140, and a processor 150. However, the device 100 is not limited thereto, and software and hardware configurations may be modified, added, or omitted within the scope obvious from the viewpoint of a person skilled in the art according to necessary operations.

The memory 110 may store data supporting, or associated with, various functions of the device 100 and a program for operation of the processor 150, may store input and output data, and may store one or more application programs or applications running on the device 100, data for operation of the device 100, one or more instructions, and an AI model. At least some of these applications may be downloaded from an external server via wireless communication.

The memory 110 may include, for example, but not limited to, at least one type of storage medium of a flash memory type, a hard disk type, a solid state disk type (SSD), a silicon disk drive type (SDD), a multimedia card micro type, a card type memory (e.g., SD or XD memory), a random access memory (RAM), a static random access memory (SRAM), a read-only memory (ROM), an electrically erasable programmable read-only storage (EEPROM), a programmable read/only memory (PROM), a magnetic memory, a magnetic disk, and an optical disc.

In addition, the memory 110 may be separate from the device 100 or may not be comprised in the device 100, and the memory 100 may be included in a database connected by wire or wirelessly. That is, the database 200 shown in FIG. 1 may include the memory 110 or be implemented as a component of the memory 110.

The communication module or communicator 120 may include one or more components configured to communicate with an external device, for example, a broadcast receiving module, a wired communication module, a wireless communication module, a shortrange communication module, and a location information module.

The wired communication module may include various wired communication modules, such as a local area network (LAN) module, a wide area network (WAN) module, or a value added network (VAN) module, as well as various cable communication modules such as a universal serial bus (USB), a high definition multimedia interface (HDMI), a digital visual interface (DVI), a restricted standard 232 (RS-232), power line communication, or a plain old telephone service (POTS).

The wireless communication module may include a wireless communication module configured to support various wireless communication methods, such as a global system for mobile communication (GSM), code division multiple access (CDMA), Wideband Code Division Multiple Access (WCDMA), universal mobile telecommunications system (UMTS), time division multiple access (TDMA), long term evolution (LTE), 4G, 5G, and 6G, in addition to a WiFi module and a wireless broadband module.

The display 130 outputs (e.g. displays) information processed in the device 100 (e.g., data input or output through an AI model and physical property prediction information). In addition, the display 130 may display execution screen information of an application program (for example, an application) driven by the device 100, or user interface (UI) and graphic user interface (GUI) information according to the execution screen information.

The input module 140 is configured to receive information from a user, and when the information is input through a user input unit, the processor 150 may control the operation of the device 100 to correspond to the input information.

As such, the input module 140 may include hardware physical keys (e.g., buttons, dome switches, jog wheels, jog switches, a touch panel, a microphone, etc. located on at least one of the front, back, and side surfaces of the device) and software touch keys. As an example, the touch key may be a virtual key, a soft key, or a visual key displayed on the touchscreen-type display 130 through software processing, or may be a touch key disposed in a portion other than the touchscreen. On the other hand, the virtual key or the visual key may be displayed on a touchscreen in various forms including, for example, but not limited to, graphics, text, icons, video, or combinations thereof.

The processor 150 may be operably connected with the memory 110 that stores an algorithm for controlling operations of components in the device 100 (including learning or execution of an AI model) or data for a program that reproduces the algorithm. The processor 140 may execute or perform the operations described above using the data stored in the memory. In this case, the memory 110 and the processor 150 may be implemented as separate chips, or may be implemented as a single integrated chip.

In addition, the processor 150 may control one or more of the above-mentioned components of the device 100 in order to implement various embodiments according to the present disclosure described below on the device 100.

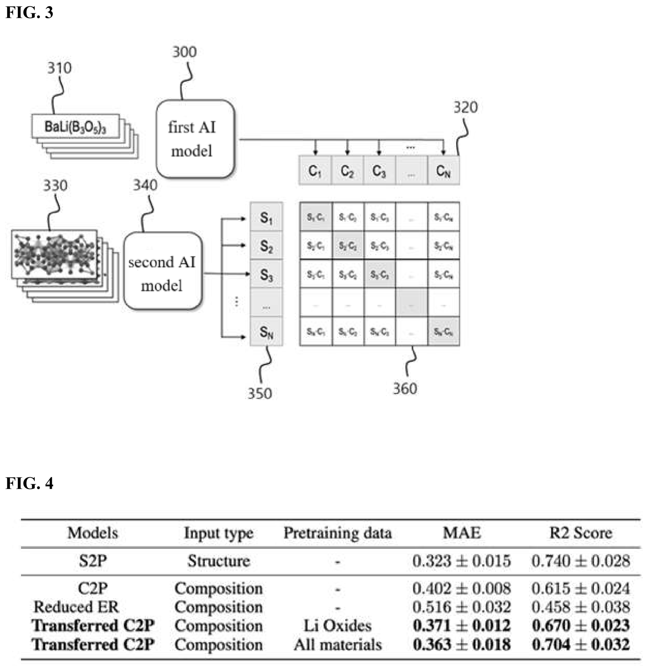

FIG. 3 is a schematic block diagram illustrating a method of learning an AI model, according to an embodiment of the present disclosure.

When the composition information 310 of a material is input to a first AI model 300, the first AI model 300 outputs feature data 320 for the input composition information 310 of the material.

Before performing the learning according to an embodiment of the present disclosure, the first AI model 300 may be an algorithm predetermined or pre-configured to output a feature vector for composition information of a material, or may be a model learned based on various information of materials (e.g. composition enthalpy, refractive index, band gap, phonon frequency, volume elastic modulus, Debye temperature, thermal conductivity, thermal expansion coefficient, decomposition enthalpy and the like). The algorithm of the first AI model 300 may be, for instance, but not limited to, a Multi-Layer Perceptron (MLP), a Graph Neural Network (GNN), a Transformer Encoder, or the like.

In an embodiment, the composition information 310 input to the first AI model 300 is of the same chemical formula as $BaLi(B_3O_5)_3$, and the output feature data 320 is a feature vector and has N values $C_1, C_2, C_3, \ldots C_N$ in N dimensions. The first AI model 300 may extract composition ratio information of each element from an input chemical formula, vectorize the information through Mat2Vec embedding, Element embedding, Fractional embedding, or the like, and then output the extracted information to a feature vector through multi head attention and Residual Network, and the output feature vector may be in a matrix format or may be a plurality of dimensions, and may select and output only some feature vectors from all of the feature vectors. When only some feature vectors are selected, the selected feature vectors may be a value representative of the compositional information of the material.

When the structure information 330 of a material is input, the second AI model 340 outputs feature data 350 for the input structure information 330 of the material. The information input to the first AI model 300 and the second AI model 340 is for the same material.

Before performing the learning according to an embodiment of the present disclosure, the second AI model 340 may be an algorithm predetermined or pre-configured for outputting a feature vector for structural information of a material, or may be a model learned based on various structural information of the material. The algorithm of the second AI model 340 may be, for example, but not limited to, a Graph Neural Network (GNN), a Crystal-Graph Convolution Neural Network (CGCNN), a Digital Message Passing Neural Network (DIMENET), or a Material Graph Network (MEGNET).

In an embodiment, the structure information 330 input to the second AI model 340 is one or more of information indicating the structure of $BaLi(B_3O_5)_3$ (a three-dimensional coordinate of each element, an interatomic distance, a molecular bond angle, a structure image, and the like), and the output feature data 350 has N values $S_1, S_2, S_3, \ldots S_N$ that are N-dimensional as a feature vector. Here, the feature vector 320 output by the first AI model 300 and the feature vector 350 output by the second AI model 340 are preferably the same dimensionality (i.e., the same N) so as to correspond to each other 1:1, although not required.

After the first AI model 300 and the second AI model 340 output the first feature vector 320 and the second feature vector 350, respectively, the first AI model 300 is learned or updated based on the second feature vector 350 value that is an output value of the second AI model 330, and the second AI model 340 is learned or updated based upon a first feature vector 320 value that is the output value of the first AI model 330. That is, the first AI model 300 and the second AI model 340 are subjected to mutual learning or contrast learning based on output values of each other, and such learning may be repeatedly performed. In still other embodiments, the first AI model 300 may be learned only based on the output value of the second AI model 340 and the second AI model 34 may not be learned based on the output values of the first AI model 30. In this embodiment, the second AI model 340 may be referred to as teaching the first AI model 300.

The first AI model 300 being learned based on the output value of the second AI model 340 may be performed by a processor of a device or a computer, and the first AI model 300 can learn to output feature data having an association with the feature data that is the output value of second AI model 340. Here, the meaning of "having an association" (including similarity) includes a situation that both data are related in some way, such as numerical values are similar or have a specific functional relationship, or are determined to be related in probability, as compared with other cases not having an association.

In an embodiment, a first vector, $C_1$, of the N first feature vectors 320 output by the first AI model 300 is compared and contrasted with a first vector, $S_1$, of the N second feature vectors 350 output by the second AI model 340, and $C_1$ adjusts the $C_1$ value so as to have similarity to $S_1$ and not to have similarity to the remaining second feature vectors, $S_2$ to $S_N$. Likewise, the values of the remaining first feature vectors 320, $C_2$ to $C_N$, are also adjusted using the remaining second feature vector 350 $S_2$ to $S_N$ in the case of the same dimension among $S_2$ to $S_N$. The first AI model 300 is then learned or updated such that the adjusted feature vectors $C_1$ to $C_N$ are output when structural information of the same material is input.

As a method of adjusting the corresponding value to be closer and more similar, the non-corresponding value to be farther and more dissimilar, the corresponding value (positive value) may be larger in size, and the non-correlated value (negative value) may be smaller in size as in the following equation. That is, the value on the left side is a positive value, and the value on the right side is a negative value, so that the positive value is greater than the negative value. A cosine similarity may be used as the score below, and methods such as Euclidean Distance, Manhattan Distance, and Minkowski Distance may be used as a method of measuring similarity between two vectors.

$$Score(f(x), f(x^+)) > Score(f(x), f(x^-))$$

Structural similarity may mean that two materials have similar crystal structures, which means that the arrangement of atoms, the symmetry of the crystals, the parameters of the unit cell, and the like are similar. Similar feature vector values for structural information result in structural similarity. On the other hand, compositional similarity may mean that two materials have a similar chemical composition, which means that the kind or proportion of atoms constituting the composition is similar. Similar feature vector values for compositional information result in compositional similarity. In some embodiments of the present disclosure, feature vectors of structural information and feature vectors of compositional information may be similar to each other.

As described above, learning the first AI model 300 to output feature data having similarity to feature data that is an output value of the second AI model 340 may be repeatedly performed.

In an embodiment, after the first AI model 300 is learned to output the adjusted feature data based on the output value of the second AI model 340, the second AI model 330 may also be learned based on the adjusted output data of the first AI model 340. That is, the second AI model 340 is trained to output feature data having an association with feature data that is an output value of the first AI model 300. In an embodiment, each of the N second feature vectors 350 output by the second AI model 340 is adjusted to have similarity with a corresponding one of the N first feature vectors 320 output by the first AI model 340, where the first feature vectors are values adjusted based on the second feature vectors according to the foregoing embodiment. The second AI model 340 is then learned or updated such that the adjusted feature vectors $S_1$ to $S_N$ are output when structural information of the same material is input.

On the other hand, in the above embodiment, it is described that the first AI model 300 is first learned based on the feature data output by the second AI model 340. Alternatively, the second AI model 330 may be learned first based upon the feature data outputted by the first AI model 300. And, the first AI model 300 is learned based on the feature data output by the second AI model 340, then the second AI model 330 is learned based on the feature data output by first AI model 300, and again, first AI model 300 may be repeatedly re-learned based on the feature data that second AI model 340 outputs, and after learning for one material, the learning may also be performed for another material. In an embodiment, the first AI model 300 and the second AI model 340 may use only lithium oxide materials or may use all kinds of materials including lithium oxide as a learning target material.

Generally, structural information of a material includes more information than composition information, and therefore the structural information is preferably used rather than the composition information in order to extract feature data of the material. However, since there is a problem that the structural information of a material may be difficult to know or obtain compared to the composition information, many studies and tests inevitably use the composition information to obtain feature data even though the composition information may show low performance.

However, the first AI model 300 according to an embodiment of the present disclosure not only receives composition information of a material and outputs feature data thereof, but also learns based on the feature data of the structure information of the material output by the second AI model 340, and thus the output of the first AI model 300 may have an association with the structure information of a material. In addition, when the first AI model 300 and the second AI model 340 repeatedly perform the learning through mutual contrast learning, the first AI model 300 may extract feature data having a closer association with the structure information of the material even though only the composition information of the material is input.

When feature data of a material is extracted as described above, if the first AI model 300 according to an embodiment of the present disclosure is used (that is, the second AI model is used only for learning of the first AI model, and then only the first AI model is used without the second AI model when the desired feature data of the material is extracted, obtained, and output), a similar level of effect as that of extracting feature data using structure information of the material may be obtained only by composition information of the material.

Fig. 4 is a table for explaining the performance of an AI model according to an embodiment of the present disclosure.

In FIG. 4, average voltage prediction performance was measured for four artificial intelligence models, such as a structure encoder (S2P) using a known DimeNet, a Reduced ER using a known Matminer and PCA (Principal Component Analysis), Transferred C2P (Pretraining data: Li Oxides) learned based on lithium oxide materials in the artificial intelligence model according to an embodiment of the present disclosure, and Transferred C2P (Pretrained data: All materials) learned based on all materials including lithium oxide in the artificial intelligence models according to the disclosure.

First, in the case of S2P, since structural information of a material is used as input data, it may be confirmed that the Mean Absolute Error (MAE) score and the R2 score are the best (the lower the MAE score and the higher the R2 score, the better the performance may be determined). On the other hand, in the case of C2P and Reduced ER, since composition information of a material is used as the input data, it may be confirmed that the MAE score and the R2 score are worse than those of S2P using the structure information as the input data. Incidentally, in the case of Transferred C2P, which is an artificial intelligence model according to an embodiment of the present disclosure, it may be confirmed that even when composition information of a material is used as input data, there is no significant difference in MAE score and R2 score compared to S2P, which uses the structural information as input data. In particular, it may be seen that models learned from all kinds of materials have better performance than models learned from only lithium oxide materials. As described above, it may be seen that using the AI model according to some embodiments of the present disclosure may achieve a similar level of effect as extracting feature data using structure information of a material even with composition information of the material.

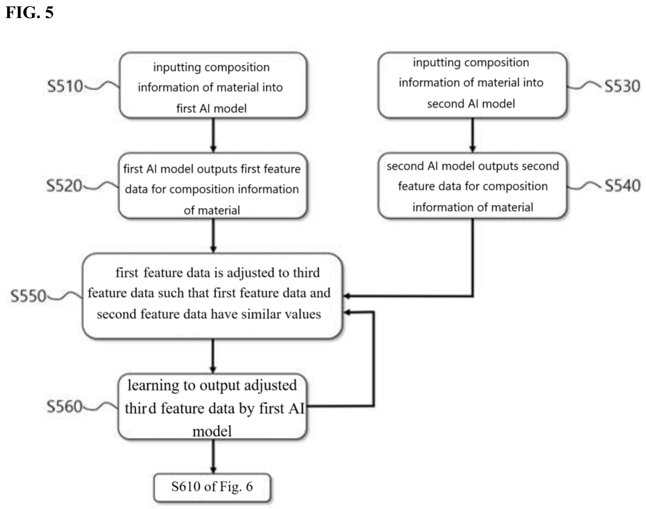

FIG. 5 is a flowchart for explaining a method of learning an AI model according to an embodiment of the present disclosure.

At operation S510, the device inputs composition information of a material into the first AI model. At operation S520, the first AI model outputs first feature data on the composition information of the material, while the device inputs structure information of the material to the second AI model at operation S530. At operation S540, the second AI model outputs second feature data on the structure information of the material.

Subsequently, at operation S550, the device adjusts the first feature data to third feature data so that the first feature data for the composition information of the material output from the first AI model and the second feature data for the structure information of the material output from the second AI model can have similar values. At operation S560, the device learns for the first AI model to output the adjusted third feature data. According to an embodiment, the device may repeat operations S550 and S560 several times, so that the adjusted third feature information and the adjusted second feature information can have similar values, and the first AI model can again output the adjusted first feature information.

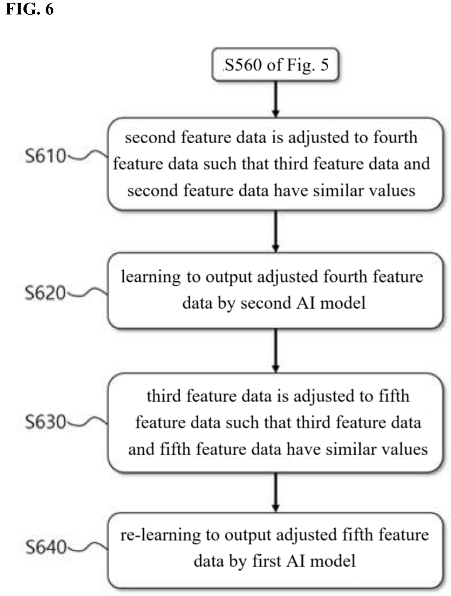

Meanwhile, in an embodiment of the present disclosure, the device may use the first AI model learned after executing up to step S560 to obtain feature data on composition information of a target material for which analysis is necessary, but may use the first AI model re-learned after undergoing an additional learning process according to an embodiment as disclosed in FIG. 6 below.

FIG. 6 is a flowchart for explaining a method of learning an AI model according to an embodiment of the present disclosure.

At operation S610, the device adjusts the second feature data to fourth feature data such that, after the first AI model is learned to output the adjusted third feature data from the composition information of the material according to the embodiment of FIG. 5, the third feature data and the second feature data output by the second AI model from the structure information of the material can have similar values. The similar values may mean values of which difference is within a predetermined allowable range. At operation S620, the second AI model is learned to output the fourth feature data adjusted in operation S610.

Subsequently, at operation S630, the third feature data is adjusted to fifth feature data so that the third feature data output by the first AI model and the fourth feature data output by a second AI model can have similar values. At operation S640, the first AI model is re-learned to output the fifth feature data adjusted in operation S630.

In the embodiment disclosed in FIG. 6, as the first AI model for outputting feature data for composition information of a material and the second AI model for outputting feature data for structural information of a material repeatedly perform mutual learning or contrast learning with each other, the first AI model may be created or configured so that structural characteristics of the material are further considered. As described above in connection with FIG. 4, the AI model according to an embodiment of the present disclosure may extract feature data that shows characteristics of a material well at a level similar to that in a case where structural information is input even though only composition information of the material is input. This effect may be more clearly confirmed when the AI model according to some embodiments of the present disclosure is used for the positive electrode material or material of the battery.

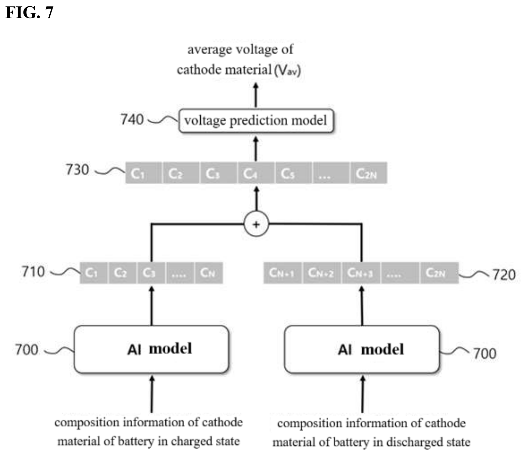

FIG. 7 is a block diagram illustrating a method of predicting or calculating an average voltage of a battery using an AI model according to an embodiment of the present disclosure.

First, positive electrode material composition information in a battery charge state and/or positive electrode material composition data in a battery discharge state (e.g., a chemical formula, a ratio or content of each element, etc.) are input to an AI model 700 according to an embodiment of the present disclosure. In the exemplary embodiment of the present disclosure, the battery may be a lithium-ion battery, and since the AI model 700 is a model configured to output feature data according to composition information of a material by adjusting the outputs feature data based on feature data of structural information according to an embodiment of the present disclosure, a difference in performance can be reduced compared to a model that outputs the feature data according to only the structural information of the material. Considering that the structural information of the battery positive electrode material is difficult to know or to obtain, the technical effect according to certain embodiments of the present disclosure having a model based on composition information of a material that a similar effect may be obtained as in a model of using structural information only may enable the related research to proceed more easily and quickly in an environment in which data is insufficient.

The AI model 700 receives positive electrode material composition information of each of battery charging and discharging states, and outputs feature data 710 and 720 for the composition information of each of battery charging and discharging states. The feature data 710 and 720 may be concatenated into final feature data 730. For example, if the characteristic data of the composition information output by the AI model 700 is 128-dimensional (i.e., 128 pieces of data), and the characteristic data of structure information is 128-dimensional (128 pieces of data), then the merged data may be 256-dimensional (256 pieces of data).

The merged feature data 730 may be input to a voltage prediction model 740 to predict an average voltage Vav of the positive electrode material. As described above, the merged feature data 730 are feature vectors output by the AI model 700 based on the composition information, and the AI model 700 may output data that may clearly indicate a feature of a material because an output value is learned to be adjusted based on the feature data of the structure information of the material, and as a result, a more accurate voltage (average voltage) may be predicted when the feature data is used as an input value of a voltage prediction model.

In addition, in the case of a battery, there is a problem that the composition information continuously changes (in particular, the content of Li changes) in the process of changing from the charged state to the discharged state of the battery, and thus it is difficult to predict the average voltage, and according to the embodiment of FIG. 7, it is possible to calculate and predict the average voltage more accurately. The voltage prediction model 740 may be prelearned based on charge and discharge state data of the lithium ion battery.

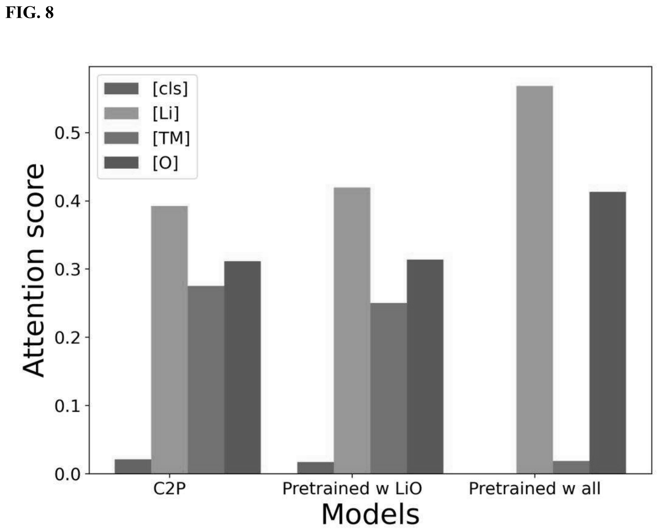

FIG. 8 is a graph for explaining the performance of a method for extracting feature data of a battery positive electrode material using an AI model according to an embodiment of the present disclosure.

FIG. 8 shows an attention score of C2P, an AI model (pretrained with LiO) learned from lithium oxide materials according to an embodiment of the present disclosure, and an AI model (retrained with all) learned from all kinds of materials according to an embodiment of the present disclosure. The attention point was calculated in a system of lithium-transition metal (TM)-oxygen structure, where TM is Ti, V, Cr, Mn, Fe, Co, Ni and Cu. Here, the attention score means how much each element is considered or referenced in the process of calculating the feature vector by the AI model, and if the attention score for the lithium (Li) element is high, it means that more lithium elements are considered or referenced than other elements in calculating the feature vector of the material containing lithium. It is important to refer to the lithium element with more weight when calculating the characteristic vector of the lithium ion battery (particularly the positive electrode material of the battery) because the lithium ion battery continuously changes the content of the lithium element as the charging and discharging states are repeated, which is an element that directly affects the battery voltage.

FIG. 8 shows four graph bars for each model, a second graph bar is for lithium (Li), a third graph bar is for a transition metal (TM), and a fourth graph bar is for an attention score of oxygen (O). As may be seen from the graph, the AI model according to an embodiment of the present disclosure has a higher attention score of Li than C2P, and in particular, it may be confirmed that the learned model based on all kinds of materials has a higher attention score of Li. Therefore, when the AI model according to an embodiment of the present disclosure is used for predicting the average voltage of a lithium ion battery, more accurate prediction and calculation can be performed.

Meanwhile, certain embodiments of the present disclosure may be implemented in the form of a recording medium storing instructions executable by a computer. The instructions may be stored in the form of program code and, when executed by a processor, may generate program modules to perform the operations of the disclosed embodiments. The recording medium may be implemented as a computer-readable recording medium.

The computer-readable recording medium includes any type of recording medium in which instructions that may be decrypted by a computer are stored. For example, Examples include a read only memory (ROM), a random access memory (RAM), a magnetic tape, a magnetic disk, a flash memory, an optical data storage device, and the like.

As described above, the disclosed embodiments are described with reference to the accompanying figures. A person of ordinary skill in the art may understand that the present disclosure may be implemented in a different form from the disclosed embodiments without changing the technical sprit or essential features of the present disclosure. The disclosed embodiments are illustrative and restrictive.

**[Explanation of Symbols]**

100: device

110: memory

120: communication module

130: display

140: input module

150: processor

200: database

300: first AI Model

340: second AI Model

700: AI model

740: voltage prediction model

The present invention further relates to the following items:

1. A system comprising:

a memory configured to store a first artificial intelligence (AI) model configured to output first feature data for composition information of a material and a second AI model configured to output second feature data for structure information of the material; and
a processor configured to execute or learn the first AI model and the second AI model, wherein the processor is configured to perform one or more of an operation of learning the first AI model based on the second feature data for the structure information of the material output by the second AI model, or an operation of learning the second AI model based on the first feature data for the composition information of the material output by the first AI model.

2. The system according to item 1, wherein: the processor is configured to learn the first AI model based on the second feature data for the structural information of the material output by the second AI model to output feature data associated with the second feature data for the structural information of the material.

3. The system according to item 2, wherein:

the first feature data and the second feature data have multiple dimensions, and
the processor is configured to learn the first AI model to output the feature data associated with the second feature data by adjusting the first feature data to third feature data to have higher similarity between the first feature data and the second feature data such that the first AI model is learned to output the third feature data adjusted from the first feature data.

4. The system according to item 3, wherein:

the third feature data and fourth feature data have multiple dimensions, and

the processor is configured to:

adjust the second feature data to the fourth feature data to have higher similarity between the second feature data and the third feature data such that the second AI model is learned to output the fourth feature data adjusted from the second feature data, and

adjust the third feature data to fifth feature data to have higher similarity between the third feature data and the fourth feature data such that the first AI model is re-learned to output the fifth feature data adjusted from the third feature data.

5. The system according to item 1, wherein:

the first AI model includes one or more of Multi-Layer Perceptron (MLP), Graph Neural Network (GNN), or Transformer Encoder, and

the second AI model includes a GNN.

6. The system according to item 1, wherein:

the material is one of multiple materials comprising lithium oxide, and

the processor is configured to learn the first AI model and the second AI model based on one of the multiple materials, and re-learn the first AI model and the second AI model based on another of multiple materials.

7. The system according to item 1, wherein: the first AI model, learned based on the second feature data for the structure information of the material output by the second AI model, is configured to receive as an input composition information of a cathode material of a battery in a charged state and/or composition information of the cathode material of the battery in a discharged state to acquire feature data related to an average voltage of the battery.

8. The system according to item 1, wherein: the feature data is a feature vector.

9. A computer-implemented method comprising:

outputting, by a processor, first feature data by inputting composition information of a material into a first artificial intelligence (AI) model stored in memory;

outputting, by the processor, second feature data by inputting structural information of the material into a second AI model stored in the memory; and

performing, by the processor, one or more of an operation of learning the first AI model based on the second feature data for the structural information of the material output by the second AI model, or an operation of learning the second AI model based on the first feature data for the composition information of the material output by the first AI model.

10. The computer-implemented method according to item 9, wherein:
the operation of the learning of the first AI model based on the second feature data for the structural information of the material output by the second AI model comprises learning the first AI model to output feature data associated with the second feature data.

11. The computer-implemented method according to item 10, wherein:

the first feature data and the second feature data have multiple dimensions, and

the learning of the first AI model to output the feature data associated with the second feature data comprises adjusting the first feature data to a third feature data to have higher similarity between the first feature data and the second feature data such that the first AI model is learned to output the third feature data adjusted from the first feature data.

12. The computer-implemented method according to item 11, wherein:

the third feature data and fourth feature data have multiple dimensions, and

the computer-implemented method further comprises:

adjusting, by the processor, the second feature data to the fourth feature data to have higher similarity between the second feature data and the third feature data such that the second AI model is learned to output the fourth feature data adjusted from the second feature data, and

adjusting, by the processor, the third feature data to fifth feature data to have higher similarity between the third feature data and the fourth feature data such that the first AI model is re-learned to output the fifth feature data adjusted from the third feature data.

13. The computer-implemented method according to item 9, further comprising:

inputting composition information of a cathode material of a battery in a charged state and/or composition information of the cathode material of the battery in a discharged state to the first AI model learned based on the second feature data for the structure information of the material output by the second AI model; and

acquiring, by the processor, feature data related to an average voltage of the battery by.

14. A non-transitory computer-readable storage medium having instructions that, when executed by one or more processors, cause the one or more processors to:

output first feature data by inputting composition information of a material into a first artificial intelligence (AI) model stored in memory;

output second feature data by inputting structural information of the material into a second AI model stored in the memory; and

perform one or more of an operation of learning the first AI model based on the second feature data for the structural information of the material outputted by the second AI model, or an operation of learning the second AI model based on the first feature data for the composition information of the material outputted by the first AI model.

**Claims**

1. A computer-implemented method for training artificial intelligence (AI) models for acquiring feature data, the method comprising the following steps in the following order:

1.1 inputting (S510) composition information of a material into a first AI model, the first AI model outputting (S520) first feature data on the composition information of the material;
1.2 inputting (S530) structural information of the material into a second AI model, the second AI model outputting (S540) second feature data on the structure information of the material;
1.3 adjusting (S550) the first feature data to third feature data such that the third feature data has values similar to the second feature data;

1.4 training (S560) the first AI model to output the third feature data;

1.5 adjusting (S610) the second feature data to fourth feature data such that the fourth feature data has values similar to the third feature data;

1.6 training (S620) the second AI model to output the fourth feature data;

1.8 adjusting (S630) the third feature data to fifth feature data such that the fifth feature data has values similar to the fourth feature data; and

1.9 re-training (S640) the first AI model to output the fifth feature data.

2. The method according to claim 1, wherein the adjusting steps are performed such that a score of a corresponding value between feature data of a same dimension is greater than a score of a non-corresponding value.

3. The method according to claim 2, wherein the score is calculated based on Cosine Similarity, and a method for measuring similarity between vectors includes one of Euclidean Distance, Manhattan Distance, or Minkowski Distance.

4. The method according to claim 1, wherein the first AI model is one of a Multi-Layer Perceptron (MLP), a Graph Neural Network (GNN), or a Transformer Encoder, and wherein the second AI model is a Graph Neural Network (GNN).

5. The method according to claim 1, wherein the first AI model and the second AI model are initially trained based on lithium oxide materials, and subsequently re-trained based on all materials including the lithium oxide materials.

6. The method according to claim 1, wherein the first feature data and the second feature data are feature vectors having a same number of dimensions.

7. A system comprising:

a memory configured to store a first artificial intelligence (AI) model configured to output feature data for composition information of a material and a second AI model configured to output feature data for structure information of the material; and
a processor configured to execute or train the first AI model and the second AI model,
wherein the processor is configured to: input composition information of a material into the first AI model to output first feature data; input structural information of the material into the second AI model to output second feature data;

adjust the first feature data to third feature data such that the third feature data has values similar to the second feature data; train the first AI model to output the third feature data; adjust the second feature data to fourth feature data such that the fourth feature data has values similar to the third feature data; train the second AI model to output the fourth feature data; adjust the third feature data to fifth feature data such that the fifth feature data has values similar to the fourth feature data; and re-train the first AI model to output the fifth feature data.

8. The system according to claim 7, wherein the processor is configured to adjust the feature data such that a score of a corresponding value between feature data of a same dimension is greater than a score of a non-corresponding value.

9. The system according to claim 8, wherein the processor is configured to calculate the score based on Cosine Similarity, and to measure similarity between vectors using one of Euclidean Distance, Manhattan Distance, or Minkowski Distance.

10. The system according to claim 7, wherein the first AI model is one of a Multi-Layer Perceptron (MLP), a Graph Neural Network (GNN), or a Transformer Encoder, and wherein the second AI model is a Graph Neural Network (GNN).

11. The system according to claim 7, wherein the processor is configured to initially train the first AI model and the second AI model based on lithium oxide materials, and subsequently re-train the models based on all materials including the lithium oxide materials.

12. The system according to claim 7, wherein the first feature data and the second feature data are feature vectors having a same number of dimensions.

**FIG. 1**

**FIG. 2**

**FIG. 3**

**FIG. 4**

| Models | Input type | Pretraining data | MAE | R2 Score |
|---|---|---|---|---|
| S2P | Structure | - | $0.323 \pm 0.015$ | $0.740 \pm 0.028$ |
| C2P | Composition | - | $0.402 \pm 0.008$ | $0.615 \pm 0.024$ |
| Reduced ER | Composition | - | $0.516 \pm 0.032$ | $0.458 \pm 0.038$ |
| **Transferred C2P** | Composition | Li Oxides | $\mathbf{0.371 \pm 0.012}$ | $\mathbf{0.670 \pm 0.023}$ |
| **Transferred C2P** | Composition | All materials | $\mathbf{0.363 \pm 0.018}$ | $\mathbf{0.704 \pm 0.032}$ |

**FIG. 5**

S510 — inputting composition information of material into first AI model

inputting composition information of material into second AI model — S530

S520 — first AI model outputs first feature data for composition information of material

second AI model outputs second feature data for composition information of material — S540

S550 — first feature data is adjusted to third feature data such that first feature data and second feature data have similar values

S560 — learning to output adjusted third feature data by first AI model

S610 of Fig. 6

**FIG. 6**

S560 of Fig. 5

S610 — second feature data is adjusted to fourth feature data such that third feature data and second feature data have similar values

S620 — learning to output adjusted fourth feature data by second AI model

S630 — third feature data is adjusted to fifth feature data such that third feature data and fifth feature data have similar values

S640 — re-learning to output adjusted fifth feature data by first AI model

**FIG. 7**

**FIG. 8**